(19) Europäisches Patentamt
European Patent Office
Office européen des brevets

(11) Veröffentlichungsnummer : **0 288 428 B1**

(12) # EUROPÄISCHE PATENTSCHRIFT

(45) Veröffentlichungstag der Patentschrift : **29.01.92 Patentblatt 92/05**

(51) Int. Cl.$^5$ : **C07C 37/08,** C07C 39/14, // C07C409/08

(21) Anmeldenummer : **88810176.3**

(22) Anmeldetag : **18.03.88**

(54) **Verfahren zur Herstellung von 2,6-Dihydroxynaphthalin.**

(30) Priorität : **24.03.87 CH 1120/87**

(43) Veröffentlichungstag der Anmeldung : **26.10.88 Patentblatt 88/43**

(45) Bekanntmachung des Hinweises auf die Patenterteilung : **29.01.92 Patentblatt 92/05**

(84) Benannte Vertragsstaaten : **BE CH DE ES FR GB IT LI NL**

(56) Entgegenhaltungen :
**EP-A- 0 032 758**
**FR-A- 1 277 033**
**US-A- 3 141 046**
**US-A- 4 262 143**

(73) Patentinhaber : **CIBA-GEIGY AG**
**Klybeckstrasse 141**
**CH-4002 Basel (CH)**

(72) Erfinder : **Clausen, Martin**
**Birchstrasse 72**
**CH-8057 Zürich (CH)**
Erfinder : **Rys, Paul, Prof.Dr.**
**In der Looren 51**
**CH-8053 Zürich (CH)**

EP 0 288 428 B1

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft ein neues, verbessertes Verfahren zur Herstellung von 2,6-Dihydroxynaphthalin durch Oxidation entsprechender 2,6-Dialkylnaphthaline und anschliessende Hydrolyse der entstandenen Dihydroperoxide.

Die Oxidation von Cumol zu Cumolhydroperoxid mit Sauerstoff in Gegenwart von Alkalimetallsalzen verzweigter Carbonsäuren wird in der US-A-3 141 056 beschrieben. Das Verfahren wird ohne Lösungsmittel bei höheren Temperaturen durchgeführt.

Aus der US-A-4 503 262 ist ferner bereits ein Verfahren zur Herstellung von 2,6-Diisopropylnaphthalindihydroperoxid bekannt. Als Katalysatoren verwendet man z.B. Oxide oder Hydroxide von Schwermetallen oder auch Schwermetallsalze organischer Säuren. Das Verfahren wird in aliphatischen Kohlenwasserstoffen als Lösungsmittel bei 50 bis 100°C durchgeführt.

Es wurde nun gefunden, dass man die Oxidation der 2,6-Dialkylnaphthaline auch ohne den Einsatz von Schwermetallkatalysatoren und zwar selektiver und mit guten Ausbeuten durchführen kann, indem man stattdessen Alkalioder Erdalkalimetallsalze organischer Carbonsäuren verwendet.

Gegenstand der vorliegenden Erfindung ist daher ein Verfahren zur Herstellung von 2,6-Dihydroxynaphthalin, welches dadurch gekennzeichnet ist, dass man 2,6-Dialkylnaphthaline, gelöst in inerten organischen Lösungsmitteln, bei Temperaturen von 50 bis 150°C mit Sauerstoff oder Sauerstoff abgebenden Verbindungen in Gegenwart eines Alkali- oder Erdalkalimetallsalzes einer organischen Carbonsäure mit 5 bis 14 Kohlenstoffatomen zu den entsprechenden 2,6-Dialkylnaphthalindihydroperoxiden oxidiert und diese anschliessend hydrolysiert.

Bei den erfindungsgemäss eingesetzten 2,6-Dialkylnaphthalinen handelt es sich um solche mit niederen Alkylsubstituenten, vorzugsweise mit 2 bis 4 und insbesondere mit 3 oder 4 Kohlenstoffatomen, in den angegebenen Positionen des Naphthalinringsystems.

Solche Alkylgruppen sind z.B. Aethyl, Propyl, Isopropyl, Butyl, Isobutyl, sek.-Butyl oder tert.-Butyl, wobei Alkylgruppen mit einem tertiären Proton in $\alpha$-Stellung zum Arylsystem, insbesondere Isopropyl und sek.-Butyl, bevorzugt sind.

Bei den im erfindungsgemässen Verfahren verwendeten Alkali- oder Erdalkalimetallsalzen organischer Carbonsäuren mit 5 bis 14 Kohlenstoffatomen handelt es sich vorzugsweise um Verbindungen der Formel

$$\left[ R_1 - \underset{\underset{R_2}{|}}{CH} - COO \right]_n M$$

worin $R_1$ $C_1$-$C_{12}$-Alkyl oder Naphthyl und $R_2$ Wasserstoff oder $C_1$-$C_9$-Alkyl ist, die Summe der Kohlenstoffatome in den Substituenten $R_1$ und $R_2$ 3 bis 12 beträgt, M Lithium, insbesondere Natrium und Kalium, sowie Magnesium, Strontium, Barium und insbesondere Calzium und n in Abhängigkeit von der Wertigkeit der Metalle M 1 oder 2 ist.

Als organische Carbonsäuren, deren Salze verwendet werden, kommen beispielsweise in Betracht: n-Valeriansäure, Capronsäure, n-Heptylsäure, Caprylsäure, Pelargonsäure, Caprinsäure, Laurinsäure, Myristinsäure, 2-Ethylbuttersäure, 4-Ethylhexansäure, 3-Ethylheptansäure, 2-Naphthylessigsäure und vorzugsweise 2-Ethylcapronsäure.

Die Salzbildung kann auch in-situ stattfinden, d.h. man gibt die organischen Carbonsäuren und in der Regel Alkalimetall- oder Erdalkalimetallhydroxide getrennt in das Reaktionsgemisch.

Bevorzugt verwendet werden die Calzium- und insbesondere die Natriumsalze oder Kaliumsalze dieser Säuren und zwar in Mengen von 0,1 bis 0,5 Gew.-%, bezogen auf das Gewicht der 2,6-Dialkylnaphthaline.

Gegebenenfalls kann man für die erfindungsgemässe Oxidationsreaktion zusätzlich sog. Reaktionsinitiatoren, wie z.B. organische Radikalbildner aus der Gruppe der Peroxide, z.B. Di-tert.-butylperoxid, oder der Azoverbindungen, z.B. Azobisisobutyronitril, einsetzen. Die bei der Oxidation entstehenden Mono- oder Dihydroperoxide (z.B. 2,6-Diisopropylnaphthalindihydroperoxid), z.B. aus einer vorgängigen Oxidation, können ebenfalls als sog. Reaktionsinitiatoren dienen.

Die Mengen dieser Initiatoren betragen etwa 0,1 bis 0,5 Gew.-%, bezogen auf das Gewicht der 2,6-Dialkylnaphthaline.

Die Oxidation wird in einem inerten organischen Lösungsmittel durchgeführt, wobei aromatische (Benzol oder halogenierte Benzole) und vorzugsweise aliphatische Kohlenwasserstoffe (Heptan, Oktan, Petroläther), insbesondere mit Siedebereichen von 60 bis 150°C, gut geeignet sind.

Als eigentliches Oxidationsmittel kann man im erfindungsgemässen Verfahren molekularen Sauerstoff oder auch Sauerstoff enthaltende Gase, wie z.B. Luft, oder Sauerstoff abspaltende Verbindungen, wie z.B. Ozon, verwenden.

Verwendet man Sauerstoff als Oxidationsmittel, so leitet man beispielsweise 0,1 bis 1 $m^3$, vorzugsweise 0,3 bis 0,6 $m^3$ dieses Gases pro kg des 2,6-Dialkylnaphthalins und pro Stunde durch das Reaktionsgemisch.

Die Reaktionszeiten liegen etwa im Bereich von 1 bis 24 Stunden. Die Reaktionstemperaturen liegen vorzugsweise im Bereich von etwa (70-80) bis 130°C, wobei die obere Begrenzung durch den Siedepunkt des verwendeten inerten Lösungsmittels gegeben ist. Der ganz besonders bevorzugte Temperaturbereich liegt bei 80 bis 110°C.

Nach Beendigung der Oxidationsreaktion wird das Reaktionsgemisch langsam auf Raumtemperatur abgekühlt. Der dabei ausfallende feinkristalline Niederschlag, der im wesentlichen 2,6-Dialkylnaphthalindi- und -monohydroperoxid enthält (durch Umkristallisieren des Niederschlags aus einem inerten Lösungsmittel kann man ein weitgehend einheitliches Dihydroperoxid erhalten) wird abgetrennt und in einem polaren Lösungsmittel, z.B. Methanol, aufgenommen und der Hydrolyse, z.B. durch Mineralsäuren, wie Salz- oder Schwefelsäure, unterworfen. Dazu wird die methanolische Lösung kurzzeitig (10 bis 40 Minuten) unter Rühren auf Temperaturen von 40 bis 65°C erhitzt.

Die Hydrolyse kann gegebenenfalls auch mit sauren Festkörperkatalysatoren, insbesondere sauren Ionenaustauschern, durchgeführt werden. Methanol wird danach abgetrennt und der Rückstand wird mit einem apolaren Lösungsmittel (z.B. Hexan, Heptan, Petrolether) versetzt und auf ca. 60°C erwärmt. Dann werden die ungelösten Teile abgetrennt, mit einem inerten Lösungsmittel (Petrolether) gewaschen und schliesslich getrocknet. Bei diesem Produkt handelt es sich um das erfindungsgemässe Verfahrensprodukt - 2,6-Dihydroxynaphthalin -, das in sehr guten Ausbeuten (praktisch quantitativ) - bezogen auf das intermediär gebildete 2,6-Dialkylnaphthalindihydroperoxid - und mit sehr guter Reinheit erhalten wird.

Das Filtrat (1), das nach dem Abtrennen des feinkristallinen Niederschlags (vor der Hydrolyse) erhalten wird, enthält im wesentlichen die Ausgangsverbindung und 2,6-Dialkylnaphthalinmonohydroperoxid. Filtrat (1) kann in eine erneute Oxidationsreaktion eingesetzt werden.

Aus dem Filtrat (2), das nach dem Abtrennen des 2,6-Dihydroxynaphthalins anfällt, kristallisiert beim Abkühlen eventuell noch vorhandenes 2-Hydroxy-6-alkylnaphthalin aus, das ebenfalls abgetrennt und getrocknet wird.

Das erfindungsgemässe Verfahren kann zyklisch und damit auch kontinuierlich geführt werden.

Zur Ueberwachung des erfindungsgemässen Verfahrens und zur Analyse der erhaltenen Produkte werden übliche analytische Methoden, wie beispielsweise die Gaschromatographie (GC) und insbesondere die Hochdruckflüssigchromatographie (HPLC), verwendet.

Die Vorteile des erfindungsgemässen Verfahrens liegen einerseits in der Vermeidung von Schwermetallkatalysatoren, andererseits in der leichten Abtrennung der erhaltenen Dihydroperoxide von den als Ausgangsverbindungen eingesetzten 2,6-Dialkylnaphthalinen, sowie auch von den als Zwischenprodukte auftretenden Monohydroperoxiden der genannten Ausgangsverbindungen.

Das erfindungsgemässe Verfahren kann wie erwähnt auch kontinuierlich durchgeführt werden.

Die verfahrensgemässen Produkte fallen in guter Ausbeute und Reinheit an. Sie sind geeignete Zwischenprodukte für die Herstellung einer Vielzahl von chemischen Produkten wie beispielsweise Farbstoffen, Kunststoffen, synthetischen Fasern, Polymeren mit flüssigkristallinen Phasen oder Pharmazeutika.

Die nachfolgenden Beispiele erläutern das erfindungsgemässe Verfahren, ohne es jedoch auf diese Beispiele zu beschränken. Teile und Prozente beziehen sich, sofern nicht anders angegeben, auf das Gewicht. Die Temperatur ist in Celsiusgraden angegeben.

## Beispiel 1

10 g 2,6-Diisopropylnaphthalin (Reinheit: 98%, gaschromatographische (GC) Analyse), gelöst in 20 ml n-Heptan, werden in einem 100 ml-Vierhalskolben, versehen mit Rührer, Thermometer, Rückflusskühler und Gaseinlassfritte, auf 80°C aufgeheizt. Unter intensivem Rühren werden in die Lösung zunächst 0,1 ml einer Lösung aus 4 Teilen 2-Ethylcapronsäure, 2 Teilen Natriumhydroxid und 5 Teilen Wasser, dann 0,02 g $\alpha,\alpha'$-Azobisisobutyronitril und schliesslich während 24 Stunden Sauerstoff in einer Menge von 80 ml/min gegeben.

Die Analyse des erhaltenen Reaktionsprodukte mittels HPLC ergibt neben unverändertem Ausgangsmaterial (2,6-Diisopropylnaphthalin) Oxidationsprodukte, die zu 91 Gew.-% aus den entsprechenden Hydroperoxiden (2,6-Diisopropylnaphthalin-Monohydroperoxid und 2,6-Diisopropylnaphthalin-Dihydroperoxid) bestehen. Das Gewichtsverhältnis von Monohydroperoxid zu Dihydroperoxid beträgt etwa 6 : 1.

Aus dem Reaktionsgemisch erhält man durch Abkühlen einen kristallinen Niederschlag, der im wesentlichen 2,6-Diisopropylnaphthalindihydroperoxid enthält. Dieser wird abfiltriert (Verarbeitung des Filtrats siehe

Beispiel 2) und dann bei Raumtemperatur mit 40 ml Methanol und tropfenweise mit 1 ml konzentrierter Salzsäure (37%ig) versetzt und die Lösung anschliessend auf 60°C erwärmt sowie 15 Minuten bei dieser Temperatur gehalten. Nach dieser Zeit werden in der Reaktionslösung keine Peroxide mehr nachgewiesen (Nachweis mittels HPLC).

Die Lösung wird unter Entfernung des Methanols eingeengt und der Rückstand in 50 ml Hexan aufgenommen und das erhaltene Lösungsmittel/Produktgemisch auf 60°C erwärmt.

Die ungelösten Teile in diesem Gemisch werden abgetrennt, mit kaltem Wasser und Petroläther (Siedebereich 110-140°C) gewaschen und getrocknet. Man erhält 0,42 g 2,6-Dihydroxynaphthalin (97%,HPLC), was bezogen auf das in der Oxidationsstufe erhaltene 2,6-Diisopropylnaphthalindihydroperoxid einer Ausbeute von 96 % entspricht.

Anstelle von 2-Ethylcapronsäure kann man in analoger Weise und mit vergleichbaren Ergebnissen auch n-Valerian-, Caprin-, Laurin- oder Myristinsäure einsetzen; anstelle von Natriumhydroxid kann auch Kaliumhydroxid verwendet werden.

Beispiel 2

Das nach dem Abtrennen des 2,6-Diisopropylnaphthalindihydroperoxids aus dem Reaktionsprodukt in Beispiel 1 erhaltene Filtrat mit einem Gehalt von

53 % 2,6-Diisopropylnaphthalin und
41,5 % 2,6-Diisopropylnaphthalinmonohydroperoxid

werden in der Apparatur wie in Beispiel 1 beschrieben auf 80°C erwärmt und mit 0,1 ml einer Lösung aus 4 Teilen 2-Ethylcapronsäure, 2 Teilen Natriumhydroxid und 5 Teilen Wasser versetzt. In diese Reaktionslösung wird dann unter Rühren Sauerstoff in einer Menge von 55 ml/min eingeleitet. Nach einer Reaktionszeit von 15 Stunden enthält die Reaktionslösung (gemäss HPLC) die folgenden Hauptkomponenten

30,5 % 2,6-Diisopropylnaphthalin
46 % 2,6-Diisopropylnaphthalinmonohydroperoxid
und 10,5 % 2,6-Diisopropylnaphthalindihydroperoxid.

Die Oxidationsprodukte bestehen zu etwa 81 Gew.-% aus den genannten Mono- bzw. Dihydroperoxiden.

Die Reaktionslösung wird mit 50 ml n-Heptan versetzt. Man kühlt auf Raumtemperatur ab und erhält einen weissen, feinkristallinen Nieder schlag, der abgetrennt wird. Er besteht aus

75 % 2,6-Diisopropylnaphthalindihydroperoxid
9 % 2,6-Diisopropylnaphthalinmonohydroperoxid
und 16 % 2-(6-Isopropyl)naphth-2-yl-propan-2-ol und
2-(6-Isopropyl)naphthyl-methylketon.

Das Filtrat enthält zu etwa 90% 2,6-Diisopropylnaphthalin und 2,6-Diisopropylnaphthalinmonohydroperoxid sowie zu etwa 1 % 2,6-Diisopropylnaphthalindihydroperoxid. Das Filtrat kann zur erneuten Autoxidation eingesetzt werden.

Der kristalline Niederschlag wird in 50 ml Methanol gelöst und tropfenweise mit 1 ml konzentrierter Salzsäure ( 37%) versetzt. Die Lösung wird dann auf 60°C aufgeheizt und 15 Minuten bei dieser Temperatur belassen. Anschliessend erfolgt die Aufarbeitung und Isolierung des 2,6-Dihydroxynaphthalins wie in Beispiel 1 beschrieben. Ausbeute: 95%, bezogen auf das in der Oxidationsstufe erhaltene 2,6-Diisopropylnaphthalindihydroperoxid.

Beispiel 3

10 g 2,6-Diisopropylnaphthalin (98%, GC) gelöst in 15 ml Oktan-Fraktion (124-128°C) werden in 100 ml Vierhalskolben, versehen mit Rührer, Thermometer, Rückflusskühler und Gaseinlassfritte auf 110°C erwärmt. Nach Zugabe von 0,1 ml einer Lösung aus 4 Teilen 2-Ethylcapronsäure, 2 Teilen Natriumhydroxid und 5 Teilen Wasser gibt man unter intensivem Rühren noch 0,1 ml Ditertiärbutylperoxid zur Reaktionslösung und leitet Sauerstoff in einer Menge von 65 ml/min ein.

Nach 6 Stunden wurde die Reaktion beendet. Die Analyse des Reaktionsprodukts durch HPLC ergibt neben unverändertem Ausgangsmaterial Oxidationsprodukte, die zu 82% aus 2,6-Diisopropylnaphthalinmono- und -dihydroperoxid bestehen, wobei ihr Gewichtsverhältnis untereinander etwa 5 : 1 beträgt.

Beim Abkühlen der Reaktionsprodukte auf Raumtemperatur kristallisiert weiss-rötliches 2,6-Diisopropylnaphthalindihydroperoxid zusammen mit wenig 2,6-Diisopropylnaphthalinmonohydroperoxid. Letzteres kann mit Oktan grösstenteils ausgewaschen werden. Das beim Abnutschen des Kristallbreis anfallende Filtrat kann nach Zusatz von 2,6-Diisopropylnaphthalin erneut in die Oxidationsreaktion eingesetzt werden. Der gereinigte Kristallbrei wird wie in Beispiel 1 beschrieben in Methanol aufgenommen und durch saure Hydrolyse zum 2,6-Dihydroxynaphthalin umgesetzt.

Die Ausbeute an 2,6-Dihydroxynaphthalin, bezogen auf 2,6-Diisopropylnaphthalindihydroperoxid, ist praktisch quantitativ.

Anstelle von 2,6-Diisopropylnaphthalin kann man mit vergleichbaren Ergebnissen auch 2,6-Di-sek.-butylnaphthalin in die Oxidationsreaktion einsetzen.

## Patentansprüche

1. Verfahren zur Herstellung von 2,6-Dihydroxynaphthalin, dadurch gekennzeichnet, dass man 2,6-Dialkylnaphthaline in einem inerten Lösungsmittel bei Temperaturen von 50 bis 150°C mit Sauerstoff oder Sauerstoff abgebenden Verbindungen in Gegenwart eines Alkali- oder Erdalkalimetallsalzes einer organischen Carbonsäure mit 5 bis 14 Kohlenstoffatomen zu den entsprechenden 2,6-Dialkylnaphthalindihydroperoxiden oxidiert und diese anschliessend hydrolysiert.

2. Verfahren nach Anspruch 1, dadurch gekennzeichnet, dass die 2,6-Dialkylnaphthaline 2 bis 4, insbesondere 3 oder 4 Kohlenstoffatome im Alkylteil enthalten.

3. Verfahren nach Anspruch 2, dadurch gekennzeichnet, dass die 2,6-Dialkylnaphthaline 2,6-Di-sek.-butyl- und insbesondere 2,6-Diisopropylnaphthaline sind.

4. Verfahren nach einem der Ansprüche 1 bis 3, dadurch gekennzeichnet, dass die Alkali- oder Erdalkalimetallsalze der organischen Carbonsäuren der Formel

$$\left[ R_1 - \underset{\displaystyle R_2}{\underset{|}{CH}} - COO \right]_n M$$

entsprechen, worin $R_1$ $C_1$-$C_{12}$-Alkyl oder Naphthyl und $R_2$ Wasserstoff oder $C_1$-$C_9$-Alkyl ist, die Summe der Kohlenstoffatome in den Substituenten $R_1$ und $R_2$ 3 bis 12 beträgt, M Lithium, insbesondere Natrium oder Kalium, sowie Magnesium, Strontium oder insbesondere Calzium und n in Abhängigkeit von der Wertigkeit der Metalle M 1 oder 2 ist.

5. Verfahren nach einem der Ansprüche 1 bis 3, dadurch gekennzeichnet, dass die Alkali- oder Erdalkalimetallsalze der organischen Carbonsäuren der Formel

$$\left[ R_1 - \underset{\displaystyle R_2}{\underset{|}{CH}} - COO \right]_n M$$

entsprechen, worin $R_1$ $C_1$-$C_{12}$-Alkyl und $R_2$ Wasserstoff oder $C_1$-$C_9$-Alkyl ist, die Summe der Kohlenstoffatome in den Substituenten $R_1$ und $R_2$ 3 bis 12 beträgt, M Lithium, insbesondere Natrium oder Kalium, sowie Magnesium, Strontium oder insbesondere Calzium und n in Abhängigkeit von der Wertigkeit der Metalle M 1 oder 2 ist.

6. Verfahren nach Anspruch 5, dadurch gekennzeichnet, dass man die Salze von n-Valeriansäure, Capronsäure, n-Heptylsäure, Caprylsäure, Perlargonsäure, Caprinsäure, Laurinsäure, Myristinsäure, 2-Ethylbuttersäure, 4-Ethylhexansäure, 3-Ethylheptansäure und vorzugsweise 2-Ethylcapronsäure, verwendet.

7. Verfahren nach einem der Ansprüche 4 bis 6, dadurch gekennzeichnet, dass die Menge der Alkali- oder Erdalkalimetallsalze der organischen Carbonsäuren 0,1 bis 0,5 Gew.-%, bezogen auf die 2,6-Dialkylnaphthaline beträgt.

8. Verfahren nach einem der Ansprüche 1 bis 3, dadurch gekennzeichnet, dass die inerten organischen Lösungsmittel aromatische oder aliphatische Kohlenwasserstoffe mit Siedepunkten im Bereich von 60 bis 150°C sind.

9. Verfahren nach einem der Ansprüche 1 bis 8, dadurch gekennzeichnet, dass man die Oxidation in Gegenwart eines zusätzlichen radikalbildenden Reaktionsinitiators durchführt.

10. Verfahren nach Anspruch 7, dadurch gekennzeichnet, dass man organische Peroxid- oder Azoverbindungen als Reaktionsinitiatoren verwendet.

11. Verfahren nach einem der Ansprüche 1 und 10, dadurch gekennzeichnet, dass die Oxidation bei Temperaturen von 70 bis 130°C durchgeführt wird.

12. Verfahren nach Anspruch 11, dadurch gekennzeichnet, dass die Oxidation bei Temperaturen von 80 bis 130°C, insbesondere bei 80 bis 110°C durchgeführt wird.

## Claims

1. A process for the preparation of 2,6-dihydroxynaphthalene, which comprises oxidizing 2,6-dialkylnaphthalenes, in an inert solvent, at temperatures from 50 to 150°C by means of oxygen or oxygen donors in the presence of an alkali metal salt or alkaline earth metal salt of an organic carboxylic acid having 5 to 14 carbon atoms, to give the corresponding 2,6-dialkylnaphthalene dihydroperoxides, and then hydrolysing the latter.

2. A process according to claim 1, wherein the 2,6-dialkylnaphthalenes contain 2 to 4, especially 3 or 4, carbon atoms in the alkyl moiety.

3. A process according to claim 2, wherein the 2,6-dialkylnaphthalenes are 2,6-di-sec-butylnaphthalenes and, especially, 2,6-diisopropylnaphthalenes.

4. A process according to any one of claims 1 to 3, wherein the alkali metal salts or alkaline earth metal salts of the organic carboxylic acids have the formula

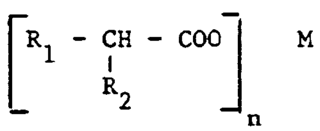

in which $R_1$ is $C_1$-$C_{12}$alkyl or naphthyl and $R_2$ is hydrogen or $C_1$-$C_9$alkyl, the sum of the carbon atoms in the substituents $R_1$ and $R_2$ is 3 to 12, M is lithium, especially sodium or potassium, and also magnesium, strontium or, in particular, calcium and n is 1 or 2, depending on the valency of the metals M.

5. A process according to any one of claims 1 to 3, wherein the alkali metal salts or alkaline earth metal salts of the organic carboxylic acids have the formula

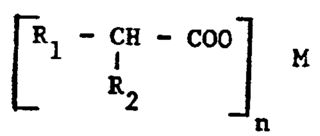

in which $R_1$ is $C_1$-$C_{12}$alkyl and $R_2$ is hydrogen or $C_1$-$C_9$alkyl, the sum of the carbon atoms in the substituents $R_1$ and $R_2$ is 3 to 12, M is lithium, especially sodium or potassium, and also magnesium, strontium or, in particular, calcium and n is 1 or 2, depending on the valency of the metals M.

6. A process according to claim 5, wherein the salts of n-valeric acid, caproic acid, n-heptanoic acid, caprylic acid, pelargonic acid, capric acid, lauric acid, myristic acid, 2-ethylbutyric acid, 4-ethylhexanoic acid, 3-ethylheptanoic acid and, preferably, 2-ethylcaproic acid are used.

7. A process according to any one of claims 4 to 6, wherein the amount of the alkali metal salts or alkaline earth metal salts of the organic carboxylic acids is 0.1 to 0.5% by weight, relative to the 2,6-dialkylnaphthalenes.

8. A process according to any one of claims 1 to 3, wherein the inert organic solvents are aromatic or aliphatic hydrocarbons having boiling points within the range from 60 to 150°C.

9. A process according to any one of claims 1 to 8, wherein the oxidation is carried out in the presence of an additional reaction initiator which forms free radicals.

10. A process according to claim 7, wherein organic peroxide or azo compounds are used as reaction initiators.

11. A process according to either of claims 1 and 10, wherein the oxidation is carried out at temperatures from 70 to 130°C.

12. A process according to claim 11, wherein the oxidation is carried out at temperatures from 80 to 130°C, particularly at 80 to 110°C.

## Revendications

1. Procédé de préparation du 2,6-dihydroxy-naphtalène, caractérisé en ce que l'on oxyde un 2,6-dialkyl-naphtalène, dans un solvant inerte, à une température de 50 à 150°C, avec de l'oxygène ou un composé libérant de l'oxygène, en présence d'un sel de métal alcalin ou alcalino-terreux d'un acide organique carboxylique comportant de 5 à 14 atomes de carbone, en le dihydroperoxyde de 2,6-dialkyl-naphtalène correspondant, et en ce qu'on hydrolyse ensuite ce dernier.

2. Procédé selon la revendication 1, caractérisé en ce que les groupes alkyles du 2,6-dialkyl-naphtalène comportent de 2 à 4 atomes de carbone et en particulier 3 ou 4 atomes de carbone.

3. Procédé selon la revendication 2, caractérisé en ce que le 2,6-dialkyl-naphtalène est du 2,6-di-s-butyl-naphtalène ou en particulier du 2,6-di-isopropyl-naphtalène.

4. Procédé selon l'une des revendications 1 à 3, caractérisé en ce que le sel de métal alcalin ou alcalino-terreux d'acide organique carboxylique correspond à la formule

$$R_1 - \underset{R_2}{CH} - COO_{\;n} \quad M$$

dans laquelle $R_1$ représente un groupe alkyle en $C_{1-12}$ ou naphtyle et $R_2$ représente un atome d'hydrogène ou un groupe alkyle en $C_{1-9}$, la somme des nombres des atomes de carbone des substituants $R_1$ et $R_2$ valant de 3 à 12, et M représente un atome de lithium, ou en particulier de sodium ou de potassium, ou encore un atome de magnésium, de strontium, ou en particulier de calcium, et n vaut 1 ou 2, en fonction de la valence du métal M.

5. Procédé selon l'une des revendications 1 à 3, caractérisé en ce que le sel de métal alcalin ou alcalino-terreux d'acide organique carboxylique correspond à la formule

$$R_1 - \underset{R_2}{CH} - COO_{\;n} \quad M$$

dans laquelle $R_1$ représente un groupe alkyle en $C_{1-12}$ et $R_2$ représente un atome d'hydrogène ou un groupe alkyle en $C_{1-9}$, la somme des nombres des atomes de carbone des substituants $R_1$ et $R_2$ valant de 3 à 12, et M représente un atome de lithium, ou en particulier de sodium ou de potassium, ou encore un atome de magnésium, de strontium, ou en particulier de calcium, et n vaut 1 ou 2, en fonction de la valence du métal M.

6. Procédé selon la revendication 5, caractérisé en ce que l'on utilise un sel de l'acide n-valérique, caproïque, n-heptylique, caprylique, perlargonique, caprique, laurique, myristique, 2-éthyl-butyrique, 4-éthyl-hexanoïque, 3-éthyl-heptanoïque, ou en particulier 2-éthyl-caproïque.

7. Procédé selon l'une des revendications 4 à 6, caractérisé en ce que la quantité de sel de métal alcalin ou alcalino-terreux d'acide organique carboxylique représente de 0,1 à 0,5 % en poids, par rapport au 2,6-dialkyl-naphtalène.

8. Procédé selon l'une des revendications 1 à 3, caractérisé en ce que le solvant organique inerte est choisi parmi les hydrocarbures, aromatiques ou aliphatiques, présentant un point d'ébullition situé dans le domaine allant de 60 à 150°C.

9. Procédé selon l'une des revendications 1 à 8, caractérisé en ce que l'on réalise l'oxydation en présence d'un amorceur de réaction, formant des radicaux et ajouté en plus.

10. Procédé selon la revendication 9, caractérisé en ce que l'on utilise, comme amorceur de réaction, un composé organique de type peroxyde ou azoïque.

11. Procédé selon l'une des revendications 1 et 10, caractérisé en ce que l'on effectue l'oxydation à une température de 70 à 130°C.

12. Procédé selon la revendication 11, caractérisé en ce que l'on effectue l'oxydation à une température de 80 à 130°C, en particulier de 80 à 110°C.